# EUROPEAN PATENT SPECIFICATION

(11) **EP 4 279 047 B1**
(45) Date of publication and mention of the grant of the patent: **09.09.2026**
(21) Application number: 22173834.7
(22) Date of filing: 17.05.2022
(51) Int. Cl.: A61F 13/514, A61F 13/537, A61F 13/539, A61F 13/58, A61F 13/62

(54) **ENVIRONMENTALLY-FRIENDLY ABSORBENT ARTICLE WITH FASTENING SYSTEM**
UMWELTFREUNDLICHER ABSORBIERENDER ARTIKEL MIT BEFESTIGUNGSSYSTEM
ARTICLE ABSORBANT SANS DANGER POUR L'ENVIRONNEMENT AVEC SYSTÈME DE FIXATION

(43) Date of publication of application: 22.11.2023
(73) Proprietor: Ontex BV, 9255 Buggenhout (BE); Ontex Group NV, 9320 Erembodegem (BE)
(72) Inventor: Idelson, Alissa, 53359 Rheinbach (DE); Reuter, Agnes Dominika, 53359 Rheinbach (DE); Weber, Ainas, 53474 Bad Neuenahr-Ahrweiler (DE)
(74) Representative: Macchetta, Andrea

(56) References cited:
- US-A1- 2017 281 428

## Description

### TECHNICAL FIELD

The disclosure relates to absorbent articles such as disposable absorbent articles, preferably selected from diapers (whether for baby or adults) free of discrete/separate frontal tape/fastener and/or landing zone.

### BACKGROUND

Examples in the literature exist wherein investigations have been carried out on effective fastener solutions for diapers.

One such early examples is described in WO 03/034966 A1. What is described is a disposable absorbent article having longitudinal side edges and transverse end edges, a first end region, and a second end region opposite of the first end region. The absorbent article comprises a liquid pervious topsheet, a liquid impervious backsheet, an absorbent core disposed between the liquid pervious topsheet and the liquid impervious backsheet, and an outer nonwoven layer disposed on an outer surface of the liquid impervious backsheet. The absorbent article further comprises a closure member. The closure member is joined adjacent to the longitudinal side edge in the first end region. The closure member comprises a hook fastening material engageable with the outer nonwoven layer for forming a closure for the absorbent article. The liquid impervious backsheet comprises landing zone graphics disposed in the second end region. The landing zone graphics are covered by the outer nonwoven layer and visible through the outer nonwoven layer. Notable disadvantages however included high cost due to the necessarily higher basis weight for the outer cover layer that acted as landing zone when applied through the entire backsheet of the diaper as well as higher environmental impact due to the generally higher amount of disposable material being generated.

Some work to overcome such shortcomings has been done and is exemplified in WO2020/074400 A1. What is described therein is a disposable diaper, particularly a baby diaper, having a cover sheet close to the body, having a back sheet away from the body, and having at least one fastening flap, the back sheet having a nonwoven layer and the fastening flap having hook regions and adhesive regions, each of which come into contact with the back sheet when in the held state. According to the description, the nonwoven layer of the back sheet is bonded in a bonding pattern consisting of pattern elements and, in the held state, the adhesive regions within the pattern elements take up an average area proportion of at least 20% relative to the total area of the particular pattern element. The particular combination of a nonwoven with a bonding pattern (i.e. a nonwoven with an embossment pattern) used as an outer cover with fasteners having a combination of hooks and adhesive and where the adhesive being arranged to occupy such a defined area of the pattern elements was found to allow for improved mechanical and chemical engagement of the fasteners directly to the outer cover without the need of a dedicated landing zone being added, and moreover allowed for the use of lower basis weight nonwovens of between 10 gsm and 20 gsm. However, the need for a bonding pattern results in a limitation as to the type of nonwovens that may be suitably used (i.e. comprising thermoplastic/synthetic fibers) that may be bonded and/or embossed. Thus although one component (i.e. the classic landing zone) is disposed of, the net overall sustainability impact is almost balanced out. Moreover, the added cost of embossed nonwovens almost balances with the saving resulting from the removal of the classic landing zone making the solution almost cost-neutral rather than a saving in overall cost.

Further prior art is disclosed in US2017/281428.

Thus, although there have already been significant advancements in this field, there still remains a need to further improve the environmental impact of absorbent articles such as diapers and yet in a cost effective manner whilst retaining high performance and good fastening of the articles in use.

### SUMMARY

The disclosure relates to a disposable absorbent article as defined in Claim 1 and Claims dependent therefrom.

### BRIEF DESCRIPTION OF THE FIGURES

**Fig. 1** Is an illustration, bottom planar view (backsheet-side), of an absorbent article according to an embodiment herein.
**Fig. 2** Is an illustration, bottom planar view (backsheet-side), of an absorbent article according to an embodiment herein.
**Fig. 3** Is an illustration, cross-section, of an absorbent article according to an embodiment herein.
**Fig.** 4 Is an illustration of a portion of a male fastener according to an embodiment herein.
**Fig. 5** Is an illustration of a portion of a male fastener according to an embodiment herein.
**Fig. 6** Is an illustration of a portion of a male fastener according to an embodiment herein.
**Fig. 7** Illustrates an exemplary process for the production of high-AUL Bio-SAP.
**Fig. 8A-B** are illustrations of a male fastener according to embodiments herein.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" or "substantially" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" or "substantially" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" or "%wt" (weight percent), here and throughout the description unless otherwise defined, refers to the relative weight of the respective component based on the overall weight of the formulation.

The use of the term "layer" can refer, but is not limited, to any type of substrate, such as a woven web, nonwoven web, films, laminates, composites, elastomeric materials, absorbent materials (such as SAP and cellulose fibers/fluff mixtures), or the like. A layer can be liquid and air permeable, permeable to air but impermeable to liquids, impermeable both to air and liquid, or the like. When used in the singular, it can have the dual meaning of a single element or a plurality of elements, such as a laminate or stacked plural sub-layers forming a common layer.

The terms "nonwoven", "nonwoven layer" or "nonwoven web" are used interchangeably to mean an engineered fibrous assembly, primarily planar, which has been given a designed level of structural integrity by physical and/or chemical means, excluding weaving, knitting or papermaking (ISO 9092:2019 definition). The directionally or randomly orientated fibers, are bonded by friction, and/or cohesion and/or adhesion. The fibers may be of natural or synthetic origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

The terms "spunlace" or "spunlace nonwoven" as used herein refer to nonwoven fabrics or materials that are made by hydroentangling webs of fibers (and/or fibers) with high energy water jets for example as basically described in Evans et al. US Patent No. 3,485,706. The webs may be made of a variety of fibers such as polyester, rayon, cellulose (cotton and wood pulp), acrylic, and other fibers as well as some blends of fibers. The fabrics may be further modified to include antistatic and antimicrobial properties, etc. by incorporation of appropriate additive materials into the fiber or fiber webs.

As used herein, the term "cellulosic" or "cellulose" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.

The term "disposable" refers to absorbent articles and/or inserts that generally are not intended to be laundered or otherwise restored or reused as absorbent articles, i.e., they are intended to be discarded after a single use and, preferably, to be recycled, composted or otherwise disposed of in an environmentally compatible manner.

The term "disposed" is used to mean that an element(s) is formed (joined and positioned) in a particular place or position as a unitary structure with other elements or as a separate element joined to another element.

The terms "interior" and "exterior" refer respectively to the location of an element that is intended to be placed against or toward the body of a wearer when an absorbent article is worn and the location of an element that is intended to be placed against or toward any clothing that is worn over the absorbent article. Synonyms for "interior" and "exterior" include, respectively, "inner" and "outer", as well as "inside" and "outside", or "body-facing" and "garment-facing". Also, when the absorbent article is oriented such that its interior faces upward, e.g., when it is laid out in preparation for setting the wearer on top of it, synonyms include "upper" and "lower" and "top" and "bottom", respectively.

The term "joined" refers to configurations whereby an element is directly secured to another element by attaching the element directly to the other element, and configurations whereby an element is indirectly secured to another element by attaching the element to intermediate member(s) which in turn are attached to the other element.

The term "lateral" or "transverse" refers to a direction running at a 90 degree angle to the longitudinal direction and when combined with the term "substantially" includes directions within ±45° of the lateral direction.

The term "longitudinal" refers to a direction running parallel to the maximum linear dimension of the article and when combined with the term "substantially" includes directions within ±45° of the longitudinal direction.

"Plant-based fibers", as used herein, includes both harvested fibers and synthetic fibers that comprise bio-based content. Harvested plant-based fibers include cellulosic matter, such as wood pulp; seed hairs, such as cotton; stem (or bast) fibers, such as flax and hemp; leaf fibers, such as sisal; and husk fibers, such as coconut. Assessment of the renewably based carbon in a material can be performed through standard test methods. Using radiocarbon and isotope ratio mass spectrometry analysis, the bio-based content of materials can be determined. ASTM International has established a standard method for assessing the bio-based content of materials. The ASTM method is designated ASTM D6866-10.The application of ASTM D6866-10 to derive a bio-based content and the analysis is performed by deriving a ratio of the amount of organic radiocarbon (14C) in an unknown sample to that of a modern reference standard. The ratio is reported as a percentage with the units "pMC" (percent modern carbon). The modern reference standard used in radiocarbon dating is a NIST (National Institute of Standards and Technology) standard with a known radiocarbon content equivalent approximately to the year AD 1950. AD 1950 was chosen since it represented a time prior to thermo-nuclear weapons testing which introduced large amounts of excess radiocarbon into the atmosphere with each explosion (termed "bomb carbon"). The AD 1950 reference represents 100 pMC.

"Bio-based content" refers to the amount of carbon from a renewable resource in a material as a percent of the mass of the total organic carbon in the material, as determined by ASTM D6866- 10, method B. In order to apply the methodology of ASTM D6866-10 to determine the bio-based content of any absorbent article or component thereof, a sample can be ground into particulates less than about 20 mesh using known grinding methods (e.g., WILEY mill), and a representative sample of suitable mass taken from the randomly mixed particles. Alternatively, if the sample is merely a layer of material, then the layer itself can be analyzed without the need for a pre-grinding step. Note that any carbon from inorganic sources such as calcium carbonate is not included in determining the bio-based content of the material.

Embodiments of the articles according to the disclosure will now be described. It is understood that technical features described in one or more embodiments maybe combined with one or more other embodiments without departing from the intention of the disclosure and without generalization therefrom, especially when such combinations are explicitly or implicitly inferred.

### THE ABSORBENT ARTICLE

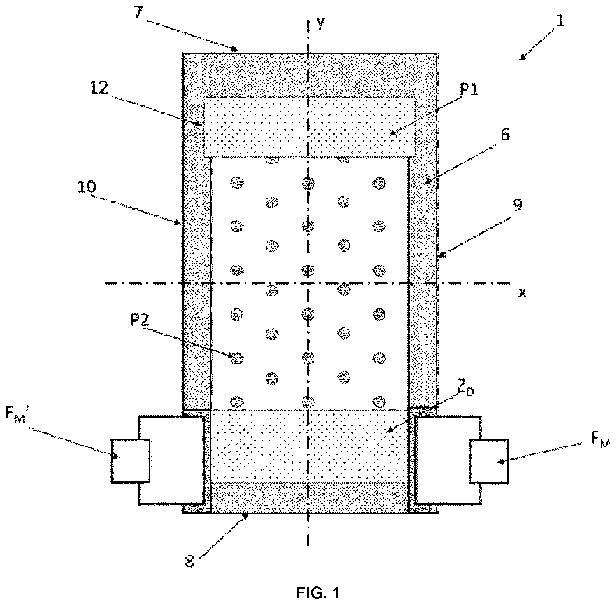

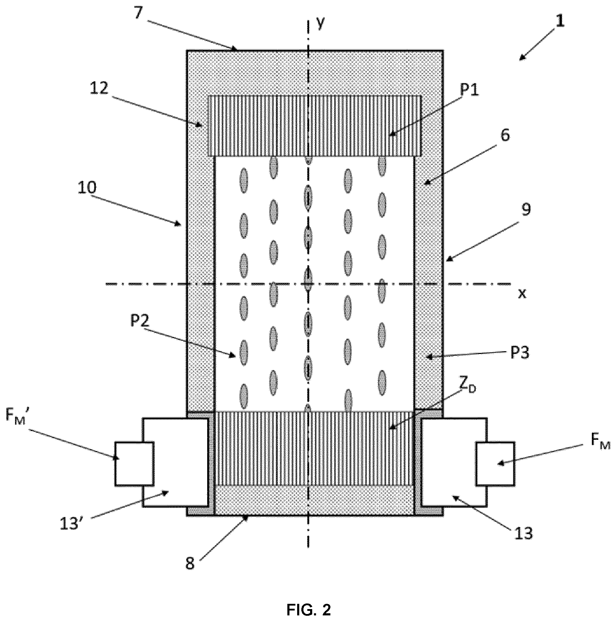

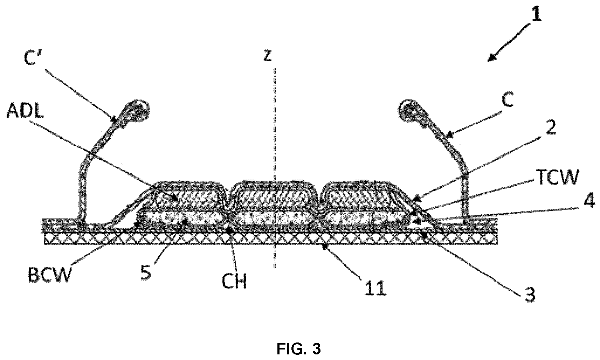

As exemplified in Figs. 1-3, absorbent articles (1) described herein comprise a liquid permeable topsheet (2), a liquid impermeable backsheet (3); and an absorbent core (4) comprising absorbent material (5) therein and being sandwiched between said topsheet (2) and backsheet (3). The topsheet (2), backsheet (3) and absorbent core (4) together generally form a chassis (6) of said article (1), wherein said chassis (6) comprises a perimeter formed by first and second transverse edges (7,8) and first and second longitudinal edges (9,10) connecting the first and second transverse edges (7,8); a longitudinal centerline (y) extending substantially parallel to said first and second longitudinal edges (9,10) and interposed therebetween such to divide said chassis (6) into a first longitudinal half between said longitudinal centerline (y) and said first longitudinal edge (9) and a second longitudinal half between said longitudinal centerline (y) and said second longitudinal edge (10); and a transverse centerline (x) extending substantially parallel to said first and second transverse edges (7,8) and interposed therebetween such to divide said chassis (6) into a first transverse half between said transverse centerline (x) and said first transverse edge (7) and a second transverse half between said transverse centerline (x) and said second transverse edge (8).

Articles herein further comprise an outer cover (11) positioned on a garment-facing side of said backsheet (3) and covering at least 75% of an area defined by said perimeter of said chassis (6); and a fastening system consisting of one or more male fasteners (F_{M},F_{M}') and a female fastening material. The female fastening material comprises a landing zone (12) arranged to receive said one or more male fasteners (F_{M},F_{M}') thereon to provide a fastening of said one or more male fasteners (F_{M},F_{M}') to said female fastening material, wherein said female fastening material comprises, preferably consists of, said outer cover (11). Advantageously added landing zone material generally used in disposable articles such as diapers may be dispensed with.

The backsheet (3) and the outer cover (11) are joined together by one or more adhesives, wherein the landing zone (12) of the outer cover (11) is joined to the backsheet (3) by a first joining pattern (P1) and the area of the outer cover (11) outboard of the landing zone (12) is joined to the backsheet (3) by a second joining pattern (P2), wherein the first joining pattern (P1) comprises a greater amount of adhesive(s) per unit area than the second joining pattern (P2) and wherein the outer cover (11) comprises a spunlace nonwoven. Without wishing to be bound by theory, spunlace nonwoven materials in particular have lower structural and mechanical integrity compared to other types of nonwovens such as spunbond, meltblown, carded and/or other synthetic based nonwovens subjected to bonding rather than hydroentanglement, yet again spunlace nonwovens are generally lofty and soft to the touch and provide excellent surface area for mechanical engagement. By ensuring a higher amount of adhesive per unit area (e.g. higher basis weight in gsm or g/m² or greater surface area coverage) is applied greater mechanical integrity of the spunlace material is provided that is better able to resist particularly to shear forces generally associated with tape fasteners. Yet again in other areas of the backsheet having a lower amount of adhesive per unit area permits to save on cost and material waste as well as providing improved overall softness.

Preferably, the amount per unit area is the basis weight in g/m² of adhesive. The first pattern (P1) thus preferably comprises a higher basis weight of adhesive compared to the second pattern (P2) and that is preferably greater than 30%, preferably at least 50%, more preferably at least 70%, even more preferably from 80% to 400%, most preferably from 95% to 300%, greater than the basis weight of adhesive in the second joining pattern (P2). Typically, "joining pattern" is intended herein as the entire area within which the referenced pattern is present (e.g. the second joining pattern P2 encompasses within its meaning the backsheet area that is subjected to such pattern i.e. the sum of the areas of the pattern itself as well as adjacent areas thereto where no other pattern is present, as shown in the figures by for example the sum of the white and shaded areas within a generally rectangular frame).

The amount per unit area may be the surface area coverage of adhesive relative to a total surface area of one or more regions of the backsheet. This may for example be inspected under UV-light and image analysis to then determine the respective surface area coverage. Advantageously this may allow for in-line automated camera inspection of the products and hence be integrated into quality control processes automatically carried out in production.

In an embodiment, the amount of adhesive(s) per unit area comprised in the first joining pattern (P1) is at least 30%, preferably at least 50%, more preferably at least 70%, even more preferably from 80% to 400%, most preferably from 95% to 300%, greater than the amount of adhesive(s) per unit area comprised in the second joining pattern (P2).

In an embodiment, the backsheet (3) and the outer cover (11) are joined together by a third bonding pattern (P3) located substantially outboard of the first and second bonding pattern (P1, P2) and along at least a portion of the perimeter of the chassis (6), wherein the third joining pattern (P3) comprises an amount of adhesive(s) per unit area that is greater than or equal to that of the first joining pattern (P1). Advantageously this allows for sealing of the cover layer at the edges in a secure manner that limits inadvertent delamination during production and/or in use.

The first bonding pattern (P1) may comprise one or more first stripes or swirls of adhesive, and wherein the second bonding pattern (P2) may comprise a plurality of second stripes or swirls of adhesive, typically wherein the first stripe(s) have an aspect ratio that is greater than that of the second stripes, the aspect ratio being defined as a ratio of a longest dimension and a smallest dimension of said stripes. Advantageously this allows for air channels or gaps to be formed between joining areas which are of greater size/volume hence improving softness to the touch though whilst this is beneficial over the majority of the surface area of the outer cover (coming to skin contact with a caregiver who places the article onto a subject) it is undesirable for the landing zone as higher risks of delamination is promoted.

In an embodiment, the first and/or second bonding pattern (P1, P2) comprises a plurality of bonding points having a circular or elliptical shape, and preferably having an aspect ratio of from about 0.1 to about 4.5, preferably from about 0.5 to about 4.0, more preferably from about 0.8 to about 3.5, even more preferably from about 0.9 to about 3.0, even more preferably from about 1 to about 2.5; and more preferably wherein the distance between neighbouring bonding points of the second bonding pattern (P2) is greater than the distance between neighbouring bonding points of the first bonding pattern (P1). Advantageously this allows for increased non-joined contact area around the bonding points promoting loftiness and softness.

Preferably, the landing zone (12) comprises a main landing zone positioned at a front portion of the chassis (6) that is closer to the first transverse edge (7) and further from the second transverse edge (8) and typically within the first transversal half of the chassis (6); and preferably a disposal-landing-zone (Z_{D}) positioned at a back portion or central portion of the chassis (6) that is closer to the second transverse edge (8) and further from the first transverse edge (7) (generally vs the front portion) and typically in the second transversal half of the chassis (6), said disposal-landing-zone (Z_{D}) adapted for receiving the one or more male fasteners (F_{M}) thereon once the absorbent article is folded or rolled-up for disposal thereof. Advantageously this allows for optimal closure of the male fasteners onto the appropriate region of the outer cover once the soiled article e.g. diaper is rolled up to contain the exudates therein and then fastened to remain in its substantially rolled-up state ready for easy disposal in a clean and non-messy manner.

In an embodiment, the outer cover (11) consists of a spunlace nonwoven, preferably substantially free of synthetic fibers. Advantageously this allows for a more natural and sustainable product especially on the largest surface of the article itself.

Preferably, the spunlace nonwoven comprises fibers consisting essentially of, preferably consisting of, cellulose fibers, preferably cotton.

Spunlace nonwovens for use as outer cover (11) herein may comprise stiffening fibers that may help to provide further resiliency to the outer cover (11)/backsheet (3) laminate. For example, as discussed hereafter, stiffening fibers may be bonded to one another via heat treatment of the outer cover (11) during production. This bonding of the stiffening fibers creates a support network which helps with resiliency and stiffness of the outer cover (11). With this in mind, the outer cover (11) may comprise from about 15% to about 60%, from about 20% to about 55%, or from about 25% to about 50% of stiffening fiber. In one specific example, the outer cover (11) may comprise from about 30% to about 35% by weight of stiffening fibers.

As mentioned previously, the outer covers of the present disclosure can provide their respective absorbent articles with a soft cushiony feel with good resiliency. Where caliper, resiliency, and a soft cushiony feel are the objective, the weight percentage of stiffening fibers may be less than or equal to the weight percentage of other fibers such as cellulose fibers.

Stiffening fibers herein may be utilized to help provide structural integrity to the outer cover (11) and especially when this is a spunlace nonwoven such is highly advantageous. The stiffening fibers can help increase structural integrity of the outer cover (11) in a machine direction and/or in a cross-machine direction which can facilitate web manipulation during processing of the outer cover (11) for incorporation into a disposable absorbent article but also later its delamination resistance (e.g. upon application of shear at fastener engagement).

Some suitable linear density values of stiffening fiber are, for example, from about 1.0 dtex to about 6 dtex, from about 1.5 dtex to about 5 dtex, or from about 2.0 dtex to about 4 dtex. In a specific example, the dtex of the stiffening fibers is about 2.2 dtex.

Examples of suitable stiffening fibers include bi-component fibers comprising polyethylene and polyethylene terephthalate components or polyethylene terephthalate and co-polyethylene terephthalate components. The components of the bi-component fiber may be arranged in a core sheath arrangement, a side by side arrangement, an eccentric core sheath arrangement, a trilobal arrangement, or the like. In one specific example, the stiffening fibers may comprise bi-component fibers having polyethylene/polyethylene terephthalate components arranged in a concentric, core-sheath arrangement where the polyethylene is the sheath. While other materials may be useful, the stiffness of polyethylene terephthalate is useful in creating a resilient structure. In contrast, the polyethylene component of the stiffening fibers can be utilized to bond to one another during heat treatment. This can help provide tensile strength to the web in both the MD and CD. Additionally, the bonding of the polyethylene component to other polyethylene components of stiffening fibers can create fixed points in the nonwoven. These fixed points can reduce the amount of fiber-to-fiber sliding which can increase the resiliency of the material.

In an embodiment, the stiffening fibers herein may comprise or consist of a material selected from the group consisting of: polylactic acid or derivatives thereof, polylactic-co-glycolic acid or derivatives thereof, polyhydroxyalkanoates or derivatives thereof, bio-polyethylene, bio-polypropylene, and mixtures thereof, preferably polylactic acid or derivatives thereof. Advantageously, this allows to not only reduce the carbon footprint of the outer cover thereby making the largest surface area of the absorbent article more environmentally friendly, but further may facilitate the recycling or composting of the used absorbent article. As discussed in more detail below these materials are typically associated with poor softness and its wide use has been somewhat limited up until now nevertheless their use as stiffening fibers herein (especially at the described lower amounts compared to e.g. cellulose fibers) may provide the optimal balance of softness, resilience and reduced carbon footprint.

One of the benefits of the stiffening fibers is that the integrated nonwoven may be heat treated post fiber entanglement e.g. post hydroentanglement of a spunlacing process. The heat treatment can provide additional structural integrity to the integrated nonwoven by forming bonds between adjacent stiffening fibers. So, where there is a higher percentage of stiffening fibers, more connection points may be created. Too many connection points can yield a much stiffer outer cover which may negatively impact comfort/softness. As such, it is advantageous to carefully control the weight percentage of the stiffening fibers when designing an absorbent article.

Regarding the heat stiffening process, any suitable temperature may be utilized. And, the suitable temperature may be impacted, in part, by the constituent chemistry of the stiffening fibers as well as by the processing fluid management layer web. For example, the outer cover may be heat stiffened at a temperature of about 132 degrees Celsius. However, it is also worth noting, that in order to provide a uniform stiffness property across the outer cover, any heating operation should be set up to provide uniform heating to the outer cover layer web. Even small variations in temperature can greatly impact the tensile strength of the outer cover.

Outer cover (11) layers for use herein may be selected from nonwovens comprising plant-based fibers wherein said plant-based fibers comprise, preferably consist of, harvested fibers other than wood pulp, such that said topsheet (2) has a bio-based content of from about 10% to about 100%, preferably from about 15% to about 100%, even more preferably from about 20% to about 100%, using ASTM D6866-10, method B, and optionally wherein said nonwoven further comprises synthetic fibers generally in an amount to provide added structural integrity such as from 10% to 50%, generally from 15% to 40%, by weight of said nonwoven.

Backsheets for use herein may be breathable and/or comprise a film, preferably polyethylene (PE) based or bio-PE based.

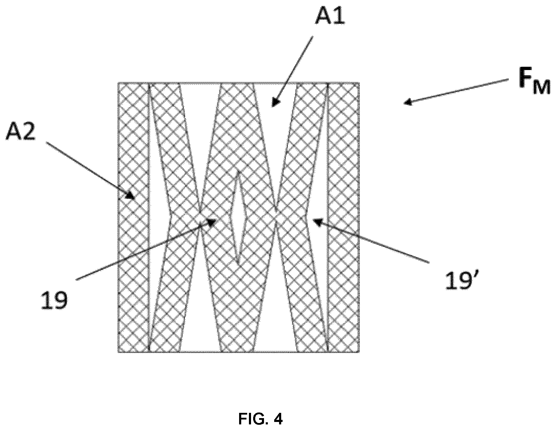

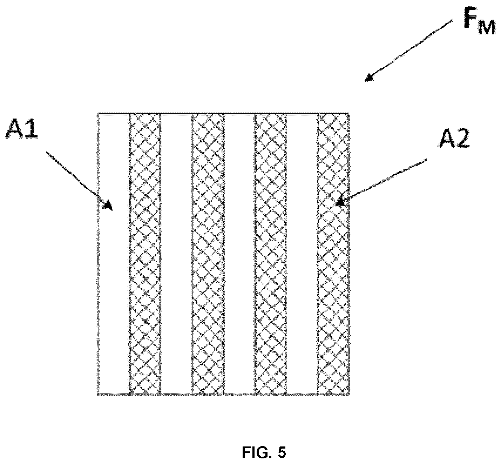

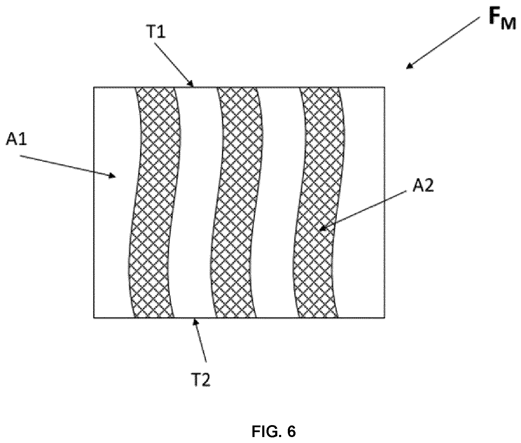

Preferably, and as exemplified in Figs. 1-6, the one or more male fasteners (F_{M},F_{M}') comprise a combination of adhesive and mechanical fastening elements preferably comprising a plurality of hooks. Advantageously having such exposed adhesive has a positive impact on peel strength and dynamic shear stress. Exposed adhesive significantly increases the initial dynamic shear strength which contributes to resistance to shear and allows to reduce the risk of delamination of the outer cover from the backsheet upon application of a shear force which would be higher if only mechanical fasteners are used, yet again adhesive alone does not provide acceptable fastening force when joined to a spunlace nonwoven.

The one or more may be joined and/or comprised on each of at least two oppositely disposed side panels (13,13') that may be elastic. Elastic side panels also referred to as elastic ears in the industry enable to increase the circumferential belly circumference of the article when worn and provide a snug fit. Elastic panels typically comprise two or more nonwoven layers and an elastic film laminated in between said nonwoven layers. Elastic side panels are typically located at the back of the article and joined to the chassis at oppositely disposed locations in the second transverse half of the chassis with at least one on each of the first and second longitudinal halves.

The at least two oppositely disposed side panels (13,13') are preferably joined to the chassis (6) at a garment-facing surface of the backsheet (3) by one or more adhesives and/or mechanical bonding wherein mechanical bonding is selected from pressure, heat, and ultrasonic bonding, and combinations thereof. When joined by adhesive alone, it is preferable that the pattern of adhesive used to join the side panels (13,13') to the backsheet (3) comprises an amount of adhesive per unit area that is greater than the second and/or third bonding pattern (P2, P3).

The one or more male fasteners (F_{M},F_{M}') comprise one or more first areas of adhesive A1 and one or more second areas of mechanical fastening elements A2 and wherein the first area(s) A1 are mutually separated by second area(s) A2. Preferably, the total area ratio Σ**A1** / Σ**A2** is at least 0.4, preferably from about 0.4 to about 2.5 (or about 2.0), more preferably between 0.4 and 1.5, more preferably from about 0.45 to about 1.0; even more preferably from about 0.5 to about 0.8, most preferably from about 0.5 to about 0.7. Advantageously this allows for improved peel strength yet limiting risks of delamination.

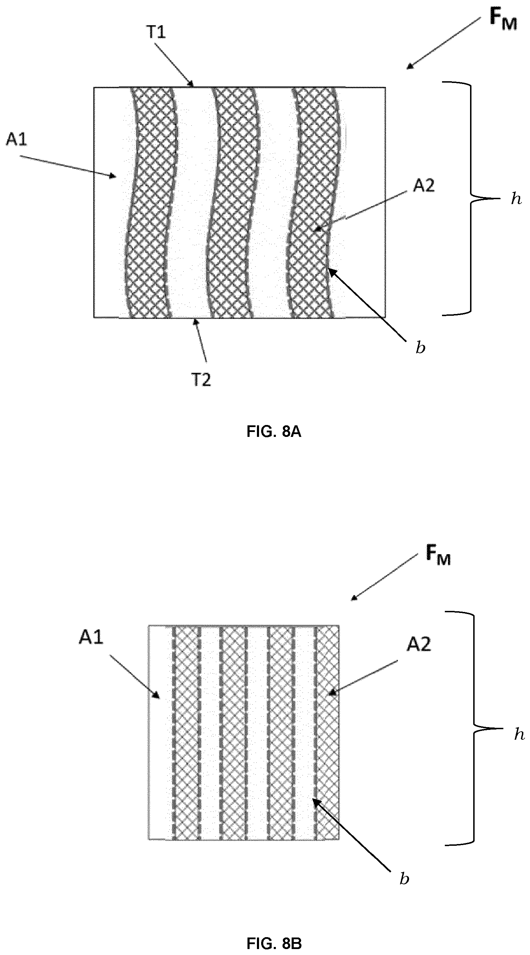

In an embodiment, and as schematically depicted in Figs. 8A-B, the one or more male fasteners (F_{M},F_{M}') comprise a cutting hight *h* and a plurality of boundaries b between mechanical fastening elements and adhesive at the border of the areas A1 and A2. The Σ*b* is preferably greater than 5h, even more preferably from 6h to 15h, even more preferably from 7h to 10h, even more preferably from 7h to less than *10h.* It has been observed that boundary edges are beneficial in providing initial peel strength, the larger the total distance of such boundaries the stronger the resistance to peel, however when too great there is an increased risk of delamination of the nonwoven and it may thus be beneficial to stay within the described preferred ranges.

Preferably, the one or more second areas of mechanical fastening elements A2 are alternatingly arranged with the one or more first areas of adhesive A1 such that said one or more second areas of mechanical fastening elements A2 are separated by one or more first areas of adhesive A1. Advantageously this allows for sequential fortifications resisting to shear.

Preferably, the one or more second areas of mechanical fastening elements A2 are disposed in a pattern such that each said second area A2, when viewed in a planar direction, forms a line-form or sinusoidal shape having a straight and/or curved perimeter.

In an embodiment, the one or more second areas of mechanical fastening elements A2 form a mesh structure (19) with one or more first areas of adhesive A1 forming one or more adhesive patches (19') substantially encircled by one or more second areas of mechanical fastening elements A2.

Preferably, the one or more first areas of adhesive A1, when viewed in a planar direction, forms a line-form or sinusoidal shape having a straight and/or curved perimeter, and wherein each of said first areas of adhesive A1 continuously extends from a first terminal position (T1) to a second terminal position (T2). Typically wherein the first terminal position (T1) and second terminal position (T2) are adjacent to respective upper and lower transverse edges of the male fastener (F_{M},F_{M}'). An advantageous peel strength can be hence achieved on spunlace nonwovens by maximising adhesive continuity along the length and/or width of the tape(s)/fastener(s).

The absorbent core (4) may comprise a core wrap enclosing the absorbent material therein. The core wrap may comprise a top core wrap layer (TCW and a bottom core wrap layer (BCW) that are joined at a periphery thereof to contain absorbent material therein and avoid leakage towards a skin of a wearer. The top core wrap layer (TCW) and a bottom core wrap layer (BCW) may be different layers or the same core wrap layer that is folded to form top and bottom layers.

Preferably, the absorbent core (4) comprises one or more attachment zones wherein the top layer (TCW) of the core wrap is adhered to the lower layer (BCW) of the core wrap such that one or more channels (CH) substantially free of absorbent material are formed, preferably inboard of a perimeter of said core (4) so that said channels (CH) do not extend to or reach said perimeter. The channels (CH) may extend from 10% to 85%, preferably from 15% to 75%, of a length of the core (4) extending in a direction substantially parallel to the longitudinal axis (y). Advantageously this allows to reduce the list of leakage as well as to provide improved cup formation when worn.

In a preferred embodiment, channels (CH) herein are longitudinally extending and cross both the transverse centerline (x) at a first crossing point and the longitudinal centerline (y) at a second crossing point, wherein the first and second crossing points are different and preferably wherein the second crossing point is positioned closer to the second transverse edge (8) than the first crossing point, and preferably wherein the second transverse edge (8) is positioned proximal to the back region of the article.

Articles herein may further comprise an acquisition distribution layer (ADL) positioned between the topsheet and the absorbent core, preferably between the topsheet and a top core wrap layer. The acquisition distribution layer may have a basis weight of from 15 gsm to 55 gsm, preferably from 18 gsm to 50 gsm, even more preferably from 19 gsm to 45 gsm. Preferably the acquisition distribution layer is selected from a carded air-through bonded nonwoven, a carded thermobonded calendered nonwoven, a spunbond nonwoven, and combinations thereof.

The articles herein may further comprise a wetness indicator that is viewable from a garment-facing side of the backsheet (3) and outer cover (11), wherein said indicator is applied to said garment-facing side of the backsheet (3). Typically wherein the wetness indicator is positioned between a garment-facing side of the core wrap and a body-facing surface of the backsheet (3), preferably positioned between a garment-facing side of the bottom/lower core wrap (BCW) and a body/skin-facing surface of the backsheet (3).

Absorbent articles herein may comprise a pair of barrier cuffs extending along the first and second longitudinal edges (C, C') and arranged to provide lateral barriers preventing exudate leakage. Typically, the barrier cuffs comprising a hydrophobic nonwoven and one or more elastics at an apex position thereof such to form standing gathers.

In an embodiment, the absorbent material comprises, preferably consists of, superabsorbent particles and/or cellulose fibers.

The absorbent cores herein may comprise at least 60%wt of superabsorbent particles, in particular at least 70%wt, preferably at least 80%wt, preferably at least 90%wt, by total weight of the core.

Preferably, the absorbent material comprises superabsorbent polymer particles preferably selected from Low-AUL Bio-SAP and High-AUL Bio-SAP as described herein. Advantageously this allows to increase the bio-based content of the disposable absorbent insert.

In an embodiment, the superabsorbent particles comprise a blend of superabsorbent particles comprising a first superabsorbent particles (SAP1) and a second superabsorbent particles (SAP2), wherein the first superabsorbent particles (SAP1) have an AUL that is greater than the AUL of the second superabsorbent particles (SAP2), and wherein the first superabsorbent particles (SAP1) have an AUL of greater than 15 g/g, according to the test method herein, preferably wherein the first superabsorbent particles (SAP1) have a particle size distribution that is greater than that of the second superabsorbent particles (SAP2). Preferably wherein at least the second superabsorbent particles (SAP2) comprises, preferably consists of, bio-based superabsorbent composite polymer particles comprising a synthetic hydrophobic polymer and a natural biopolymer, more preferably composed of a composite polymer comprising styrene maleic acid copolymer and a biopolymer of animal or vegetal origin; or non-composite polymer particles selected from polyacrylic acid, sodium salt, crosslinked, partly neutralized superabsorbent particles.

Preferably the second superabsorbent particles (SAP2) comprises, preferably consists of, Low-AUL Bio-SAP. "Low-AUL Bio-SAPs" means that they generally have AUL (as measured according to the test method herein) of less than 20 g/g, typically less than 15 g/g, and typically belong to the following classes: (a) materials consisting only of crosslinked biopolymers; (b) materials consisting only of crosslinked synthetic polymers; (c) composite materials consisting of synthetic polymers and biopolymers in certain variations: in inter crosslinking type connection with or without chemical agents; graft type; intercomplexate type and interpenetrate type. Copolymers such as styrene maleic acid are typically used in copolymerization processes for achieving Low-AUL Bio-SAP. Other exemplary sources include alpha-1,3 glucan, starch, starch and/or sodium salts, sugar and derivatives. Exemplary commercially available Low-AUL Bio-SAPs for use herein include: SAPs derived from charged-modified starches as sold from TETHIS 5237 Capital Blvd.; Raleigh, NC 27616, USA and further exemplified in US20200054782 A1 herein incorporated by reference; SAPs comprising a 100% acrylic acid co-acrylamide with little to no cross link shell, and the like all generally having AUL of less than 20 g/g according to the test method herein.

Preferably, the first superabsorbent particles (SAP1) are free of Low-AUL Bio-SAP. Advantageously this limits rewet drawbacks as described herein.

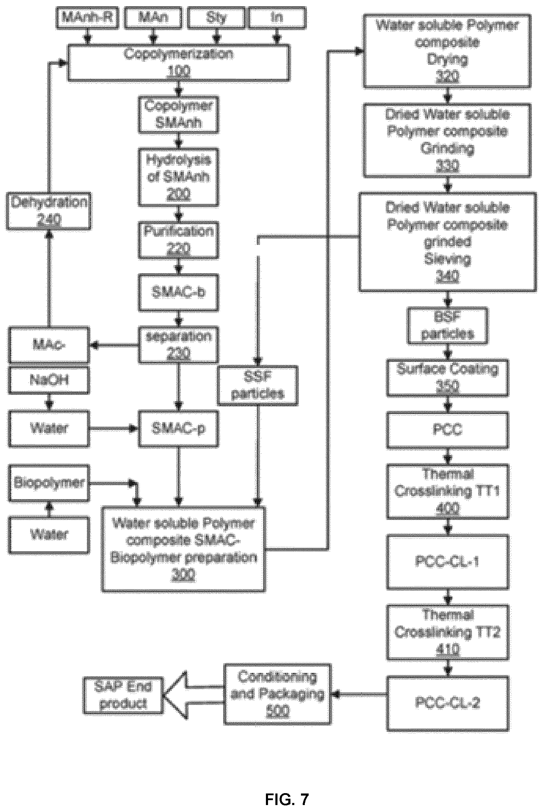

In an embodiment, the SAP1 is a High-AUL Bio-SAP "composite polymer" (i.e. a biodegradable superabsorbent composite polymer having an AUL of greater than 15 g/g, preferably greater than 20 g/g, as will be described in more detail herein below). The composite polymer may comprise a synthetic hydrophobic polymer and a natural biopolymer. More specifically, the composite polymer may comprise styrene maleic acid copolymer and a biopolymer of animal or vegetal origin in conformity with the technological flow chart presented in Fig. 7. Styrene maleic acid copolymer is preferably in salt form, more preferably in the form of monovalent cation salt. Alternatively, the High-AUL Bio-SAP, although less preferred, may comprise "non-composite polymers" and rather be selected from certified biomass superabsorbent polymers having AUL of greater than 15 g/g, preferably greater than 20 g/g, and typically certified by REDcert2 (https://www.redcert.org/images/SP_RC%C2%B2_Biomass-balanced_products_V1.0.pdf ). An example may be a polyacrylic acid, sodium salt, crosslinked, partly neutralized SAP from up to 100% allocated biomass feedstock such as commercially available HySorb^{®} B 6600MB manufactured and sold by BASF SE, Ludwigshafen, Germany.

In an embodiment of the High-AUL Bio-SAP "composite polymer", the styrene moiety in the synthetic polymer can be replaced by other hydrophobic moieties. Exemplary synthetic polymers are: poly(maleic anhydride-co- methyl vinyl ether) (Gantrez), poly(vinyl chloride-co-maleic acid) and poly[(maleic anhydride)- alt-(vinyl acetate).

The term "composite" as herein used refers to a polymeric substance that a) is formed from at least two polymers with different macromolecular chemical structure; and b) the resulting composite is a unique entity that does not separate spontaneously to its components during application. It is understood that the term "composite" may include other substances such as drugs, stimulators, inhibitors, odorants, emollients, plasticizer and others.

The term "anionic" refers to a polymeric composite generating in aqueous media a negative electrochemical potential as the result of the presence in its structure of some free acid functional groups capable of dissociating into anions.

In an embodiment described in Fig. 7, the production process starts with the synthesis of copolymer (styrene-alt-maleic anhydride) by bulk co-polymerization using an excess of about 60-90% of maleic anhydride relative to styrene, whereas maleic anhydride works also as a solvent (operation 100).

Copolymerization is typically executed in Sigma-type mixer machines named Hermetic machines in order to be able to work at high pressures e.g. not exceeding 10 bar or in vacuum conditions (less than IOmbar) with double mantle as well with arms equipped with a heating - cooling system.

The copolymerization process for the production of a superabsorbent polymer typically uses styrene monomer stabilized with organic compounds that inhibit the process of homopolymerization during storage and transportation. Such inhibitors are for example substances such as : amino derivatives, thiols derivatives, and hydroxyl derivates as for example (2-hydroxypropyl)-ethylene diamine compounds, 4-tert-butylcatechol and others) being preferred 4-tert-butylcatechol in proportion of 0.002- 0.008% to the monomer, more preferably is 0.003- 0.007% to the styrene and most preferably 0.004-0.006% to the styrene and the molar fraction of styrene in the reaction mass is 0.05-0.08, preferably 0.1-0.15 and more preferably 0.18-0.21.

The copolymerization may also contain maleic anhydride that is either fresh maleic anhydride MAnh or recovered maleic anhydride MAnh-R that is recycled from a previous batch as schematically showed in Fig. 7. and the amount of fresh maleic anhydride MAnh relative to recovered maleic anhydride MAnh-R is about 10 - 40% (dry basis).

For the copolymerization, further customary agents may be used such as peroxides, azo compounds etc., which form free radicals by thermal decomposition, while the quantity of initiator is 0.05- 0.15 %, preferably 0.07-0.009% and most preferably 0.08-0.12% to a double quantity of styrene adopted for copolymerization.

Next, it may follow the conversion of styrene-alt maleic anhydride copolymer to styrene-alt maleic acid copolymer by hydrolysis with water (process 200) which is done in the same equipment where copolymerization has been made. Conversion of styrene-alt maleic anhydride copolymer to styrene-alt maleic acid copolymer by hydrolysis using a quantity of water higher than the one stoichiometrical required for total hydrolysis (Ws) with an excess which represents 2-3% versus to the stoichiometrical value, preferably in excess of 4-5% to the stoichiometrical water and most preferably 5-10% in excess to the stoichiometrical water.

The total quantity of water necessary to styrene and maleic anhydride copolymer's hydrolysis is typically inserted in the mass of reaction in two steps from which 50% is in the form of 0.005N hydrochloric acid solution and the rest as non-acidulated water.

The acidulated water is typically inserted into the reaction mass in two portions at a mass reaction's temperature that not exceeding 60°C at 15 minutes intervals between each dosage, and the quantity of non-acidulated water is inserted into the mass of reaction also in two portions, from which the first is after 60 minutes from the last portion of acidulated water, then is inserted the last portion of non-acidulated water and mixing of reaction mass for 45-60 minutes in cooling conditions of 35-40°C. Reaction mass resulted after hydrolysis is a wet solid in form of a powdery mass of white colour with a value of bulk density of 0.6-0.8 g/cm3. Further, mass of reaction that resulted after copolymerization and hydrolysis (which is a blend of styrene maleic acid copolymer - SMAC and free maleic acid -MAC, which contains traces amounts of non-reacted styrene and stabilizer for styrene as well as traces of hydrochloric acid) is transferred to perform the purification process of styrene maleic acid copolymer (process 220).

The purification process which represents the extraction of free maleic acid fraction from SMAC polymer mass is typically done in an equipment such as tank-type with mixing in which over reaction mass resulted after hydrolysis of synthetic copolymer is added a quantity of deionized water for purification [Wp] that represents a quantity correlated to the wet mass of reactions (RM) in accordance with the relationship Wp= 2 * RM or Wp= 5 * RM, preferably Wp= 3 * RM.

Purification generally consists of 3-5 extraction stages followed each time by filtration. The number of extraction and filtration operations are established so that the content of the free carboxylic groups found in polymer of SMAC to be between 0.00909-0.0095 mole/gram, preferably between 0.0091-0.0094 mole/gram and most preferably between 0.0092- 0.0093 mole/gram.

The extraction in fact preferably occurs at temperature of 60°C for 30 minutes and each filtration (process 230) is done with known equipment as press filter or Nuce filter. All solutions resulted from filtering are collected into a tank of supernatant in order to process the maleic acid which it contain.

**The** processing of maleic acid water solution to recovery of maleic anhydride preferably consists of the following operations: a) concentration of maleic acid solution through reverse osmosis; b) spray drying of maleic acid concentrated solution when resulted maleic acid powder and water; c) conversion of maleic acid powder to maleic anhydride by thermal-vacuum dehydration (with technological parameters modified than those mentioned in US Pat No.4,414,398 when in the end is obtained a material called recovered maleic anhydride (MAnh-R). **The** purified filtrate of SMAC polymer is typically collected in an equipment as Sigma mixer type in order to be processed with biopolymers to obtain the water soluble composite polymer containing synthetic SMAC polymer and biopolymer [WSPC] (process 300).

**The** preparation process of polymeric composite [WSPC] containing SMAC and a biopolymer of animal or vegetal origin is preferably preceded by other operations as follows: a) Preparation of a base solution is obtained by dissolution of a solid hydroxide compound in water to 40% by weight, whereas examples of preferred base hydroxide compounds are sodium hydroxide, lithium hydroxide, potassium hydroxide, ammonium hydroxide, preferably sodium hydroxide; b) transformation of styrene maleic acid copolymer obtained in step 230 into styrene-maleic acid monovalent cation salt by neutralization with base solution prepared in a) above in order to obtain a solution of copolymer salt with concentration higher than 25% preferably higher than 35 % and most preferably higher than 50%. Neutralization of the SMAC copolymer is 48-58%, preferably 50-56% and most preferably 52-54%; c) preparation of the biopolymer (as gelatin, albumin, casein, soy, guar or starch, preferably is gelatin) as water solution of 40% by weight in water; d) preparation of polymeric composite is done by treating the solution of styrene maleic acid salt with biopolymer solution at temperature of 55-75°C for 30 minutes.

**The** amount of biopolymer relative to copolymer in the composite is preferably from 4-6 % (dry basis), preferably 8-10 % (dry basis) and most preferably 12-14 % (dry basis).

**The** blending of the composite mass typically continues during 4-5 hours until the polymeric mass is transformed from the viscous solution into a partially dried granular mass with a moisture content not higher than 20%. This partially dried polymeric composite material WSPC in granular form is typically subjected to a supplementary drying (process 320) at temperatures of preferably 75-85°C on conveyor belt type or Rotary type in order to obtain final drying of until the moisture content is less than 14%, preferably less than 12% and most preferably less than 8%.

Further steps of drying, grinding and sieving are preferably carried out (process 330 + process 340) to obtain two types of solid phases called herein large solid phase (LSP) with granulometric distribution higher than 100 microns, preferably of 100 -850 microns that corresponds to be used in the manufacture of diapers and a small solid phase (SSF) with granulometric distribution up to 100 microns. The small solid phase SSF is re-used in the preparation of the next new batch of SAP that comes into the process 300.

The large solid phase LSP may be exposed to post-treatment surface coating processes using known chemical substances as: glycerin ethylene glycol, propylene glycol or polyether hydroxyl with properties of biodegradability. Examples of preferred coating materials are hydroxyl polyether, more preferably polyethylene glycol - PEG 200 used at a rate of 0.2-2% by weight (dry basis) to LSP, preferably at a rate of 0.5-1.5% by weight and most preferably at a rate of 0.8-1.2% by weight to LSP.

Surface coating may be applied using equipment like powder coating machines at temperatures of generally 30-70°C, preferably at temperatures of 35-65°C and most preferably at temperatures of 40-60°C for 30 -90 minutes, preferably for 40-75 minutes and most preferably for 50-60 minutes. As a result of this process is resulted the material called Polymer Composite Coated Treated- PCC (operation 350).

The obtained material after surface coating (Polymer Composite Coated) may be subdue to a thermal treatment called first thermal treatment (TT1) (operation 400) consisting in warming of particles mass in hot air with temperature of 90-I40°C for 30-150 minutes, preferably with temperatures of 100-135°C for 45-120 minutes and most preferably by bulk thermal crosslinking at temperatures of 110-120°C, for 60-90 minute using equipment of conveyor belt type or shaking rotary type when is resulted an intermediary material called Polymer Composite Coated first crosslinked (PCC-CL-I).

The material PCC-CL-I may be subdue to a new thermal treatment (TT2) (operation 410) in hot air with temperature of 120-150°C for 5-30 minutes, preferably at temperatures of 125-145°C for 10-25 minutes and most preferably to temperature of 120-140°C for 15-20 minute in the same type of equipment used for TT1 and is obtained the Polymer Composite Coated second crosslinked (PCC-CL-2). The material (PCC-CL-2) may then be conditioned for 24 hours in an atmosphere in which the air has a moisture content of 65% and temperature of 20°C and then is packaged in sealed polyethylene bags (operation 500).

The material resulting after conditioning is a biodegradable SAP with AUL higher than 20g/g in aqueous solution of 0.9% NaCl at pressure of 0.9 psi, which represents the end product of the manufacturing process which is the object of the present invention, i.e. the "High-AUL biodegradable superabsorbent composite polymer" referred to herein.

Reference is made to co-pending application EP21152698.3 for further examples of Low-AUL Bio-SAP and High-AUL Bio-SAPs that may be used in absorbent cores herein, with particular reference to Example 1.

In an embodiment, the first superabsorbent particles (SAP1) are comprised at a level of less than 80%wt, preferably from 10%wt to 32%wt; and the second superabsorbent particles (SAP2) are comprised at a level of at least 20%wt, preferably from 25%wt to 100%wt, more preferably from 30% to 90%, even more preferably from 35% to 80%, even more preferably from 40% to 70%, even more preferably from 45% to 68%, by total weight of superabsorbent particles.

Preferably, the AUL ratio AULSAP1/AULSAP2) of the first superabsorbent particles (SAP1) and second superabsorbent particles (SAP2) is greater than 1.4, preferably greater than 1.5, more preferably from 1.6 to 5, even more preferably from 1.7 to 3. Advantageously, this allows to ensure the right balance of reduced rewet and fast liquid absorption especially on the lower layer and optimal performance under load.

In an embodiment, the first superabsorbent particles (SAP1) have a lower absorption speed (typically according to the vortex method herein) than the second superabsorbent particles (SAP2). Preferably, the vortex time (according to the vortex method herein) of SAP1 is more than 50 seconds, preferably from 60 seconds to 90 seconds. Preferably, the absorption speed of SAP2 is less than 45 seconds, preferably less than 40 seconds, even more preferably from 15 seconds to 35 seconds.

### TEST METHODS

### AUL (Absorbency Under Load, 0.7 psi)

Absorbency Under Load is determined similarly to the absorption under pressure test method No. 442.2-02 recommended by EDANA (European Disposables and Nonwovens Association), except that for each example the actual sample having the particle size distribution reported in the example is measured.

The measuring cell for determining AUL 0.7 psi is a Plexiglas cylinder 60 mm in internal diameter and 50 mm in height. Adhesively attached to its underside is a stainless steel sieve bottom having a mesh size of 36 µm. The measuring cell further includes a plastic plate having a diameter of 59 mm and a weight which can be placed in the measuring cell together with the plastic plate. The weight of the plastic plate and the weight together weigh 1345 g. AUL 0.7 psi is determined by determining the weight of the empty Plexiglas cylinder and of the plastic plate and recording it as WO. Then 0.900 +/- 0.005 g of water-absorbing polymer or material (particle size distribution 150 - 800 µm or as specifically reported in the examples which follow) is weighed into the Plexiglas cylinder and distributed very uniformly over the stainless steel sieve bottom. The plastic plate is then carefully placed in the Plexiglas cylinder, the entire unit is weighed and the weight is recorded as Wa. The weight is then placed on the plastic plate in the Plexiglas cylinder. A ceramic filter plate 120 mm in diameter, 10 mm in height and 0 in porosity (Duran, from Schott) is then placed in the middle of the Petri dish 200 mm in diameter and 30 mm in height and sufficient 0.9% by weight sodium chloride solution is introduced for the surface of the liquid to be level with the filter plate surface without the surface of the filter plate being wetted. A round filter paper 90 mm in diameter and < 20 µm in pore size (S&S 589 Schwarzband from Schleicher & Schüll) is subsequently placed on the ceramic plate. The Plexiglas cylinder holding the material or polymer is then placed with the plastic plate and weight on top of the filter paper and left there for 60 minutes. At the end of this period, the complete unit is taken out of the Petri dish from the filter paper and then the weight is removed from the Plexiglas cylinder. The Plexiglas cylinder holding swollen water-absorbing material or polymer is weighed out together with the plastic plate and the weight is recorded as Wb.

Absorbency under load (AUL) is calculated as follows: $AUL 0.7 psig / g = Wb - Wa / Wa - W 0$

AUL 0.3 psi and 0.5 psi are measured similarly at the appropriate lower pressure.

### Absorption speed (Vortex) measurement:

The vortex test measures the amount of time in seconds required for 2 grams of a superabsorbent material to close a vortex created by stirring 50 milliliters of saline solution at 600 revolutions per minute on a magnetic stir plate. The time it takes for the vortex to close (that the surface of the fluid becomes flat meaning that in the beginning, the centrifugal force that is caused by the rotation of the fluid creates a "coning-in" in the surface, but when the gelling of the SAP proceeds the viscosity of the fluid increases so that the depth of the indentation decreases until it's finally substantially flat) is an indication of the free swell absorbing rate of the superabsorbent material.

Equipment and materials:
1. Beaker, 100 ml.
2. Programmable magnetic stir plate, capable of providing 600 revolutions per minute.
3. Magnetic stir bar without rings, 7.9 mm × 32 mm, Teflon covered.
4. Stopwatch.
5. Balance, accurate to ± 0.01 g.
6. Saline solution, 0.9%.
7. Weighing paper.
8. Room with standard condition atmosphere: Temp = 23°C ± 1°C and Relative $Humidity = 50 % \pm 2 % .$

Test procedure:
1. Measure 50 g ± 0.01 g of saline solution into the 100 ml beaker.
2. Place the magnetic stir bar into the beaker.
3. Program the magnetic stir plate to 600 revolutions per minute.
4. Place the beaker on the center of the magnetic stir plate such that the magnetic stir bar is activated. The bottom of the vortex should be near the top of the stir bar.
5. Weigh out 2 g ± 0.01 g of the superabsorbent material to be tested on weighing paper.
6. While the saline solution is being stirred, pour the superabsorbent material to be tested into the saline solution and start the stopwatch. The superabsorbent material to be tested should be added to the saline solution between the center of the vortex and the side of the beaker.
7. Stop the stopwatch when the surface of the saline solution becomes flat, and record the time.
8. The time, recorded in seconds, is reported as the Vortex Time.

It is supposed that the present invention is not restricted to any form of realization described previously and that some modifications can be added to the presented embodiments without reappraisal of the appended claims.

## Claims

1. A disposable absorbent article (1) comprising:
a liquid permeable topsheet (2);
a liquid impermeable backsheet (3); and
an absorbent core (4) comprising absorbent material (5) therein and being sandwiched between said topsheet (2) and backsheet (3);
the topsheet (2), backsheet (3) and absorbent core (4) together forming a chassis (6) of said article (1), wherein said chassis (6) comprises a perimeter formed by first and second transverse edges (7,8) and first and second longitudinal edges (9,10) connecting the first and second transverse edges (7,8); a longitudinal centerline (y) extending substantially parallel to said first and second longitudinal edges (9,10) and interposed therebetween such to divide said chassis (6) into a first longitudinal half between said longitudinal centerline (y) and said first longitudinal edge (9) and a second longitudinal half between said longitudinal centerline (y) and said second longitudinal edge (10); and a transverse centerline (x) extending substantially parallel to said first and second transverse edges (7,8) and interposed therebetween such to divide said chassis (6) into a first transverse half between said transverse centerline (x) and said first transverse edge (7) and a second transverse half between said transverse centerline (x) and said second transverse edge (8);
wherein said article further comprises an outer cover (11) positioned on a garment-facing side of said backsheet (3) and covering at least 75% of an area defined by said perimeter of said chassis (6); and a fastening system consisting of one or more male fasteners (F_{M}, F_{M}') and a female fastening material;
and wherein the female fastening material comprises a landing zone (12) arranged to receive said one or more male fasteners (F_{M},F_{M}') thereon to provide a fastening of said one or more male fasteners (F_{M},F_{M}') to said female fastening material, wherein said female fastening material comprises, preferably consists of, said outer cover (11);
**characterised in that** the backsheet (3) and the outer cover (11) are joined together by one or more adhesives, wherein the landing zone (12) of the outer cover (11) is joined to the backsheet (3) by a first joining pattern (P1) and the area of the outer cover (11) outboard of the landing zone (12) is joined to the backsheet (3) by a second joining pattern (P2), wherein the first joining pattern (P1) comprises a greater amount of adhesive(s) per unit area than the second joining pattern (P2) and **in that** the outer cover (11) comprises a spunlace nonwoven, and wherein the one or more male fasteners (F_{M},F_{M}') comprise one or more first areas of adhesive A1 and one or more second areas of mechanical fastening elements A2 and wherein the first area(s) A1 are mutually separated by second area(s) A2.

2. An absorbent article (1) according to Claim 1 wherein the outer cover (11) consists of a spunlace nonwoven, preferably substantially free of synthetic fibers.

3. An absorbent article (1) according to any of the preceding Claims wherein the one or more male fasteners (F_{M},F_{M}') comprise a combination of adhesive and mechanical fastening elements preferably comprising a plurality of hooks.

4. An absorbent article (1) according to any of the preceding Claims wherein the amount of adhesive(s) per unit area comprised in the first joining pattern (P1) is at least 30%, preferably at least 50%, more preferably at least 70%, even more preferably from 80% to 400%, most preferably from 95% to 300%, greater than the amount of adhesive(s) per unit area comprised in the second joining pattern (P2).

5. An absorbent article (1) according to any of the preceding Claims wherein the total area ratio Σ**A1** / Σ**A2** is at least 0.4, preferably from about 0.4 to about 2.0, more preferably between 0.4 and 1.5, more preferably from about 0.45 to about 1.0; even more preferably from about 0.5 to about 0.8, most preferably from about 0.5 to about 0.7.

6. An absorbent article (1) according to any of the preceding Claims wherein the one or more second areas of mechanical fastening elements A2 are alternatingly arranged with the one or more first areas of adhesive A1 such that said one or more second areas of mechanical fastening elements A2 are separated by one or more first areas of adhesive A1.

7. An absorbent article (1) according to any of the preceding Claims wherein the one or more second areas of mechanical fastening elements A2 are disposed in a pattern such that each said second area A2, when viewed in a planar direction, forms a line-form or sinusoidal shape having a straight and/or curved perimeter.

8. An absorbent article (1) according to any of the preceding Claims wherein the one or more second areas of mechanical fastening elements A2 form a mesh structure (19) with one or more first areas of adhesive A1 forming one or more adhesive patches (19') substantially encircled by one or more second areas of mechanical fastening elements A2.

9. An absorbent article (1) according to any of the preceding Claims wherein the one or more first areas of adhesive A1, when viewed in a planar direction, forms a line-form or sinusoidal shape having a straight and/or curved perimeter, and wherein each of said first areas of adhesive A1 continuously extends from a first terminal position (T1) to a second terminal position (T2).

10. An absorbent article (1) according to any of the preceding Claims wherein the backsheet (3) and the outer cover (11) are joined together by a third bonding pattern (P3) located substantially outboard of the first and second bonding pattern (P1, P2) and along at least a portion of the perimeter of the chassis (6), wherein the third joining pattern (P3) comprises an amount of adhesive(s) per unit area that is greater than or equal to that of the first joining pattern (P1).

11. An absorbent article (1) according to any of the preceding Claims wherein the first bonding pattern (P1) comprises one or more first stripes or swirls of adhesive, and wherein the second bonding pattern (P2) comprises a plurality of second stripes or swirls of adhesive, wherein the first stripe(s) have an aspect ratio that is greater than that of the second stripes, the aspect ratio being defined as a ratio of a longest dimension and a smallest dimension of said stripes.

12. An absorbent article (1) according to any of the preceding Claims wherein the spunlace nonwoven comprises fibers consisting essentially of, preferably consisting of, cellulose fibers, preferably cotton.

13. An absorbent article (1) according to any of the preceding Claims wherein the first and/or second bonding pattern (P1, P2) comprises a plurality of bonding points having a circular or elliptical shape, and preferably having an aspect ratio of from 0.1 to 4.5, and more preferably wherein the distance between neighbouring bonding points of the second bonding pattern (P2) is greater than the distance between neighbouring bonding points of the first bonding pattern (P1).

14. An absorbent article (1) according to any of the preceding Claims wherein the landing zone (12) comprises a main landing zone positioned at a front portion of the chassis (6) that is closer to the first transverse edge (7) and further from the second transverse edge (8); and preferably a disposal-landing-zone (Z_{D}) positioned at a back portion or central portion of the chassis (6) that is closer to the second transverse edge (8) and further from the first transverse edge (7) said disposal-landing-zone (Z_{D}) adapted for receiving the one or more male fasteners (F_{M}) thereon once the absorbent article is folded or rolled-up for disposal thereof.

15. An absorbent article (1) according to any of the preceding Claims wherein the spunlace nonwoven comprises stiffening fibers, and wherein the nonwoven comprises a plurality of bonding areas wherein said bonding areas comprise heat fused stiffening fibers, preferably wherein said heat fused stiffening fibers together form a network preferably arranged to support hydroentangled cellulose fibers comprised by said nonwoven.

## Patentansprüche

1. Absorbierender Einwegartikel (1), umfassend:
eine flüssigkeitsdurchlässige Oberschicht (2);
eine flüssigkeitsundurchlässige Unterschicht (3); und
einen absorbierenden Kern (4), umfassend absorbierendes Material (5) darin, und der zwischen der Oberschicht (2) und der Unterschicht (3) eingeschoben ist;
wobei die Oberschicht (2), die Unterschicht (3) und der absorbierende Kern (4) zusammen einen Körper (6) des Artikels (1) ausbilden, wobei der Körper (6) einen Umfang umfasst, der durch eine erste und eine zweite Querkante (7,8) und eine erste und eine zweite Längskante (9, 10), die die erste und die zweite Querkante (7, 8) verbinden, ausgebildet wird; eine Längsmittellinie (y), die sich im Wesentlichen parallel zu der ersten und der zweiten Längskante (9, 10) erstreckt und derart dazwischen eingefügt ist, dass der Körper (6) in eine erste Längshälfte zwischen der Längsmittellinie (y) und der ersten Längskante (9) und eine zweite Längshälfte zwischen der Längsmittellinie (y) und der zweiten Längskante (10) geteilt ist; und eine Quermittellinie (x), die sich im Wesentlichen parallel zu der ersten und der zweiten Querkante (7, 8) erstreckt und derart dazwischen eingefügt ist, dass der Körper (6) in eine erste Querhälfte zwischen der Quermittellinie (x) und der ersten Querkante (7) und eine zweite Querhälfte zwischen der Quermittellinie (x) und der zweiten Querkante (8) geteilt ist;
wobei der Artikel ferner eine äußere Abdeckung (11) umfasst, die auf einer kleidungszugewandten Seite der Unterschicht (3) positioniert ist und mindestens 75 % einer durch den Umfang des Körpers (6) definierten Fläche abdeckt; und ein Befestigungssystem, das aus einem oder mehreren männlichen Befestigungselementen (F_{M}, F_{M}') und einem weiblichen Befestigungsmaterial besteht;
und wobei das weibliche Befestigungsmaterial einen Aufnahmebereich (12) umfasst, der angeordnet ist, um ein oder mehrere männliche Befestigungselemente (F_{M}, F_{M}') darauf zu empfangen, um eine Befestigung des einen oder der mehreren männlichen Befestigungselemente (F_{M}, F_{M}') an dem weiblichen Befestigungsmaterial bereitzustellen, wobei das weibliche Befestigungsmaterial die äußere Abdeckung (11) umfasst, vorzugsweise daraus besteht;
**dadurch gekennzeichnet, dass** die Unterschicht (3) und die äußere Abdeckung (11) durch einen oder mehrere Klebstoffe miteinander verbunden sind, wobei der Aufnahmebereich (12) der äußeren Abdeckung (11) mit der Unterschicht (3) durch ein erstes Verbindungsmuster (P1) verbunden ist und die Fläche der äußeren Abdeckung (11) außerhalb des Aufnahmebereichs (12) mit der Unterschicht (3) durch ein zweites Verbindungsmuster (P2) verbunden ist, wobei das erste Verbindungsmuster (P1) eine größere Menge an Klebstoff(en) pro Flächeneinheit als das zweite Verbindungsmuster (P2) umfasst, und **dadurch, dass** die äußere Abdeckung (11) ein Spunlace-Vlies umfasst, und wobei das eine oder die mehreren männlichen Befestigungselemente (F_{M}, F_{M}') eine oder mehrere erste Flächen von Klebstoff **A1** und eine oder mehrere zweite Flächen von mechanischen Befestigungselementen **A2** umfassen und wobei die erste(n) Fläche(n) **A1** durch (eine) zweite Fläche(n) **A2** voneinander getrennt sind.

2. Absorbierender Artikel (1) nach Anspruch 1, wobei die äußere Abdeckung (11) aus einem Spunlace-Vlies besteht, das vorzugsweise im Wesentlichen frei von synthetischen Fasern ist.

3. Absorbierender Artikel (1) nach einem der vorstehenden Ansprüche, wobei das eine oder die mehreren männlichen Befestigungselemente (F_{M}, F_{M}') eine Kombination aus Klebstoff und mechanischen Befestigungselementen umfassen, vorzugsweise umfassend eine Vielzahl von Haken.

4. Absorbierender Artikel (1) nach einem der vorstehenden Ansprüche, wobei die Menge an Klebstoff(en) pro Flächeneinheit, die in dem ersten Verbindungsmuster (P1) umfasst ist, mindestens 30 %, vorzugsweise mindestens 50 %, mehr bevorzugt mindestens 70 %, noch mehr bevorzugt von 80 % bis 400 %, am meisten bevorzugt von 95 % bis 300 %, größer als die Menge an Klebstoff(en) pro Flächeneinheit, die in dem zweiten Verbindungsmuster (P2) umfasst ist, ist.

5. Absorbierender Artikel (1) nach einem der vorstehenden Ansprüche, wobei das Gesamtflächenverhältnis Σ**A1** / Σ**A2** mindestens 0,4 beträgt, vorzugsweise von etwa 0,4 bis etwa 2,0, mehr bevorzugt von etwa 0,4 bis etwa 1,5, mehr bevorzugt von etwa 0,45 bis etwa 1,0; noch mehr bevorzugt von etwa 0,5 bis etwa 0,8, am meisten bevorzugt von etwa 0,5 bis etwa 0,7.

6. Absorbierender Artikel (1) nach einem der vorstehenden Ansprüche, wobei die eine oder die mehreren zweiten Flächen von mechanischen Befestigungselementen **A2** abwechselnd mit der einen oder den mehreren ersten Flächen aus Klebstoff **A1** derart angeordnet sind, dass die eine oder die mehreren zweiten Flächen aus mechanischen Befestigungselementen **A2** durch eine oder mehrere erste Flächen aus Klebstoff **A1** getrennt sind.

7. Absorbierender Artikel (1) nach einem der vorstehenden Ansprüche, wobei die eine oder die mehreren zweiten Flächen von mechanischen Befestigungselementen **A2** in einem Muster derart eingerichtet sind, dass jede zweite Fläche **A2**, wenn in einer planaren Richtung betrachtet, eine linienförmige oder sinusförmige Form, die einen geraden und/oder gekrümmten Umfang aufweist, ausbildet.

8. Absorbierender Artikel (1) nach einem der vorstehenden Ansprüche, wobei die eine oder die mehreren zweiten Flächen von mechanischen Befestigungselementen **A2** eine Netzstruktur (19) ausbilden, wobei eine oder mehrere erste Flächen von Klebstoff **A1** einen oder mehrere Klebstoffflecken (19') ausbilden, die im Wesentlichen durch eine oder mehrere zweiten Fläche von mechanischen Befestigungselementen **A2** umkreist sind.

9. Absorbierender Artikel (1) nach einem der vorstehenden Ansprüche, wobei die eine oder die mehreren ersten Flächen aus Klebstoff **A1**, wenn in einer planaren Richtung betrachtet, eine linienförmige oder sinusförmige Form, die einen geraden und/oder gekrümmten Umfang aufweist, ausbilden, und wobei jede der ersten Flächen aus Klebstoff **A1** sich kontinuierlich von einer ersten Endposition (T1) zu einer zweiten Endposition (T2) erstreckt.

10. Absorbierender Artikel (1) nach einem der vorstehenden Ansprüche, wobei die Unterschicht (3) und die äußere Abdeckung (11) durch ein drittes Bindungsmuster (P3) miteinander verbunden sind, das im Wesentlichen außerhalb des ersten und des zweiten Bindungsmusters (P1, P2) und entlang mindestens eines Abschnitts des Umfangs des Körpers (6) gelegen ist, wobei das dritte Verbindungsmuster (P3) eine Menge an Klebstoff(en) pro Flächeneinheit umfasst, die größer als oder gleich derjenigen des ersten Verbindungsmusters (P1) ist.

11. Absorbierender Artikel (1) nach einem der vorstehenden Ansprüche, wobei das erste Bindungsmuster (P1) einen oder mehrere erste Streifen oder Wirbel aus Klebstoff umfasst und wobei das zweite Bindungsmuster (P2) eine Vielzahl von zweiten Streifen oder Wirbeln aus Klebstoff umfasst, wobei der/die erste Streifen ein Seitenverhältnis aufweisen, das größer als das der zweiten Streifen ist, wobei das Seitenverhältnis als ein Verhältnis einer längsten Abmessung und einer kleinsten Abmessung der Streifen definiert ist.

12. Absorbierender Artikel (1) nach einem der vorstehenden Ansprüche, wobei das Spunlace-Vlies Fasern, im Wesentlichen bestehend aus, vorzugsweise bestehend aus Zellulosefasern, vorzugsweise Baumwolle, umfasst.

13. Absorbierender Artikel (1) nach einem der vorstehenden Ansprüche, wobei das erste und/oder das zweite Bindungsmuster (P1, P2) eine Vielzahl von Bindungspunkten umfasst, die eine kreisförmige oder elliptische Form aufweisen, und vorzugsweise ein Seitenverhältnis von 0,1 bis 4,5 aufweisen, und mehr bevorzugt wobei der Abstand zwischen benachbarten Bindungspunkten des zweiten Bindungsmusters (P2) größer als der Abstand zwischen benachbarten Bindungspunkten des ersten Bindungsmusters (P1) ist.

14. Absorbierender Artikel (1) nach einem der vorstehenden Ansprüche, wobei der Aufnahmebereich (12) einen Hauptaufnahmebereich umfasst, der an einem vorderen Abschnitt des Körpers (6) positioniert ist, der näher zu der ersten Querkante (7) und weiter von der zweiten Querkante (8) entfernt ist; und vorzugsweise einen Entsorgungsaufnahmebereich (Z_{D}), der an einem hinteren Abschnitt oder mittleren Abschnitt des Körpers (6) positioniert ist, der näher zu der zweiten Querkante (8) und weiter von der ersten Querkante (7) entfernt ist, wobei der Entsorgungsaufnahmebereich (Z_{D}) zum Empfangen des einen oder der mehreren männlichen Befestigungselemente (F_{M}) darauf angepasst ist, sobald der absorbierende Artikel für dessen Entsorgung gefaltet oder gerollt ist.

15. Absorbierender Artikel (1) nach einem der vorstehenden Ansprüche, wobei das Spunlace-Vlies Versteifungsfasern umfasst, und wobei das Vlies eine Vielzahl von Bindungsbereichen umfasst, wobei die Bindungsbereiche wärmeverschmolzene Versteifungsfasern umfassen, vorzugsweise wobei die wärmeverschmolzenen Versteifungsfasern zusammen ein Netzwerk ausbilden, das vorzugsweise angeordnet ist, um hydroverschlungene Zellulosefasern zu unterstützen, die durch das Vlies umfasst sind.

## Revendications

1. Article absorbant jetable (1) comprenant :
une feuille de dessus perméable aux liquides (2) ;
une feuille de fond (3) imperméable aux liquides ; et
une âme absorbante (4) comprenant un matériau absorbant (5) à l'intérieur de celle-ci et étant placée en sandwich entre ladite feuille de dessus (2) et ladite feuille de fond (3) ;
la feuille de dessus (2), la feuille de fond (3) et l'âme absorbante (4) formant ensemble un châssis (6) dudit article (1), dans lequel ledit châssis (6) comprend un périmètre formé par des premier et second bords transversaux (7, 8) et des premier et second bords longitudinaux (9,10) reliant les premier et second bords transversaux (7, 8) ; une ligne médiane longitudinale (y) s'étendant sensiblement parallèle auxdits premier et second bords longitudinaux (9, 10) et interposée entre ceux-ci de façon à diviser ledit châssis (6) en une première moitié longitudinale entre ladite ligne médiane longitudinale (y) et ledit premier bord longitudinal (9) et une seconde moitié longitudinale entre ladite ligne médiane longitudinale (y) et ledit second bord longitudinal (10) ; et une ligne médiane transversale (x) s'étendant sensiblement parallèle auxdits premier et second bords transversaux (7, 8) et interposée entre ceux-ci de façon à diviser ledit châssis (6) en une première moitié transversale entre ladite ligne médiane transversale (x) et ledit premier bord transversal (7) et une seconde moitié transversale entre ladite ligne médiane transversale (x) et ledit second bord transversal (8) ;
dans lequel ledit article comprend en outre une couverture externe (11) positionnée sur un côté faisant face vers le vêtement de ladite feuille de fond (3) et couvrant au moins 75 % d'une zone définie par ledit périmètre dudit châssis (6) ; et un système de fixation constitué d'une ou plusieurs fixations mâles (F_{M}, F_{M}') et d'un matériau de fixation femelle ;
et dans lequel le matériau de fixation femelle comprend une zone de réception (12) agencée pour recevoir ledit ou lesdites fixations mâles (F_{M}, F_{M}') sur celle-ci pour fournir une fixation de ladite ou desdites fixations mâles (F_{M}, F_{M}') audit matériau de fixation femelle, dans lequel ledit matériau de fixation femelle comprend, de préférence est constitué de, ladite couverture externe (11) ;
**caractérisé en ce que** la feuille de fond (3) et la couverture externe (11) sont réunies par un ou plusieurs adhésifs, dans lequel la zone de réception (12) de la couverture externe (11) est jointe à la feuille de fond (3) par un premier motif de jonction (P1) et la zone de la couverture externe (11) à l'extérieur de la zone de réception (12) est jointe à la feuille de fond (3) par un deuxième motif de jonction (P2), dans lequel le premier motif de jonction (P1) comprend une plus grande quantité d'adhésif(s) par surface unitaire que le deuxième motif de jonction (P2) et **en ce que** la couverture externe (11) comprend un non-tissé lacé par filage, et dans lequel la ou les fixations mâles (F_{M}, F_{M}') comprennent une ou plusieurs premières zones d'adhésif **A1** et une ou plusieurs secondes zones d'éléments de fixation mécaniques **A2** et dans lequel la ou les premières zones **A1** sont mutuellement séparées par une ou des secondes zones **A2**.

2. Article absorbant (1) selon la revendication 1 dans lequel la couverture externe (11) est constituée d'un non-tissé lacé par filage, de préférence sensiblement dépourvu de fibres synthétiques.

3. Article absorbant (1) selon l'une quelconque des revendications précédentes dans lequel la ou les fixations mâles (F_{M}, F_{M}') comprennent une combinaison d'éléments de fixation adhésifs et mécaniques comprenant de préférence une pluralité de crochets.

4. Article absorbant (1) selon l'une quelconque des revendications précédentes dans lequel la quantité d'adhésif(s) par surface unitaire comprise dans le premier motif de jonction (P1) est au moins 30 %, de préférence au moins 50 %, plus préférablement au moins 70 %, même plus préférablement de 80 % à 400 %, le plus préférablement de 95 % à 300 %, supérieure à la quantité d'adhésif(s) par surface unitaire comprise dans le deuxième motif de jonction (P2).

5. Article absorbant (1) selon l'une quelconque des revendications précédentes dans lequel le rapport surfacique total Σ**A1** / Σ**A2** est d'au moins 0,4, de préférence d'environ 0,4 à environ 2,0, plus préférablement entre 0,4 et 1,5, plus préférablement d'environ 0,45 à environ 1,0 ; encore plus préférablement d'environ 0,5 à environ 0,8, le plus préférablement d'environ 0,5 à environ 0,7.

6. Article absorbant (1) selon l'une quelconque des revendications précédentes dans lequel la ou les secondes zones d'éléments de fixation mécaniques **A2** sont agencées en alternance avec la ou les premières zones d'adhésif **A1** de telle sorte que ladite ou lesdites secondes zones d'éléments de fixation mécaniques **A2** sont séparées par une ou plusieurs premières zones d'adhésif **A1.**

7. Article absorbant (1) selon l'une quelconque des revendications précédentes, dans lequel la ou les secondes zones d'éléments de fixation mécaniques **A2** sont disposées dans un motif de telle sorte que chaque seconde zone **A2** précitée, lorsqu'elle est vue dans une direction plane, forme une forme de ligne ou une forme sinusoïdale ayant un périmètre droit et/ou incurvé.

8. Article absorbant (1) selon l'une quelconque des revendications précédentes dans lequel la ou les secondes zones d'éléments de fixation mécaniques **A2** forment une structure de treillis (19) avec une ou plusieurs premières zones d'adhésif **A1** formant un ou plusieurs timbres adhésifs (19') sensiblement encerclés par une ou plusieurs secondes zones d'éléments de fixation mécaniques **A2**.

9. Article absorbant (1) selon l'une quelconque des revendications précédentes, dans lequel la ou les premières zones d'adhésif **A1**, lorsqu'elles sont vues dans une direction plane, forment une forme de ligne ou une forme sinusoïdale ayant un périmètre droit et/ou incurvé, et dans lequel chacune desdites premières zones d'adhésif **A1** s'étend de manière continue d'une première position terminale (T1) à une seconde position terminale (T2).

10. Article absorbant (1) selon l'une quelconque des revendications précédentes dans lequel la feuille de fond (3) et la couverture externe (11) sont réunies par un troisième motif de liaison (P3) localisé sensiblement à l'extérieur des premier et deuxième motifs de liaison (P1, P2) et le long d'au moins une partie du périmètre du châssis (6), dans lequel le troisième motif de jonction (P3) comprend une quantité d'adhésif(s) par surface unitaire qui est supérieure ou égale à celle du premier motif de jonction (P1).

11. Article absorbant (1) selon l'une quelconque des revendications précédentes dans lequel le premier motif de liaison (P1) comprend une ou plusieurs premières bandes ou volutes d'adhésif, et dans lequel le deuxième motif de liaison (P2) comprend une pluralité de secondes bandes ou volutes d'adhésif, dans lequel la ou les premières bandes ont un rapport d'aspect qui est supérieur à celui des secondes bandes, le rapport d'aspect étant défini en tant que rapport d'une dimension la plus longue et d'une dimension la plus petite desdites bandes.

12. Article absorbant (1) selon l'une quelconque des revendications précédentes dans lequel le non-tissé lacé par filage comprend des fibres constituées sensiblement de, de préférence constituées de, fibres de cellulose, de préférence du coton.

13. Article absorbant (1) selon l'une quelconque des revendications précédentes dans lequel les premier et/ou deuxième motifs de liaison (P1, P2) comprennent une pluralité de points de liaison ayant une forme circulaire ou elliptique, et ayant de préférence un rapport d'aspect allant de 0,1 à 4,5, et plus préférablement dans lequel la distance entre des points de liaison voisins du deuxième motif de liaison (P2) est supérieure à la distance entre des points de liaison voisins du premier motif de liaison (P1).

14. Article absorbant (1) selon l'une quelconque des revendications précédentes, dans lequel la zone de réception (12) comprend une zone de réception principale positionnée au niveau d'une partie avant du châssis (6) qui est plus proche du premier bord transversal (7) et plus éloignée du second bord transversal (8) ; et de préférence une zone de réception pour élimination (Z_{D}) positionnée au niveau d'une partie arrière ou d'une partie centrale du châssis (6) qui est plus près du second bord transversal (8) et plus loin du premier bord transversal (7) ladite zone de réception pour élimination (Z_{D}) étant conçue pour recevoir la ou les fixations mâles (F_{M}) sur celle-ci une fois que l'article absorbant est plié ou enroulé pour élimination de celui-ci.

15. Article absorbant (1) selon l'une quelconque des revendications précédentes dans lequel le non-tissé lacé par filage comprend des fibres de raidissement, et dans lequel le non-tissé comprend une pluralité de zones de liaison dans lequel lesdites zones de liaison comprennent des fibres de raidissement fusionnées thermiquement, de préférence dans lequel lesdites fibres de raidissement fusionnées thermiquement forment ensemble un réseau agencé de préférence pour supporter des fibres de cellulose enchevêtrées par voie hydraulique comprises par ledit non-tissé.
